# EUROPEAN PATENT APPLICATION

(11) **EP 3 943 063 A1**
(43) Date of publication of application: **26.01.2022**
(21) Application number: 21186525.8
(22) Date of filing: 19.07.2021
(51) Int. Cl.: A61K 8/34, A61K 8/73, A61Q 11/00

(54) **COMPOSITION FOR USE IN THE PREVENTION OF DENTAL CARIES**

(30) Priority: 20.07.2020 IT 202000017527
(71) Applicant: Logidex S.r.l., 10132 Torino (IT); Berta, Giovanni Nicolao, 10087 Valperga (TO) (IT)
(72) Inventor: BERTA, Giovanni Nicolao, I-10087 Valperga (TO) (IT); ROMANO, Federica, I-10129 Torino (IT); DE FABIANIS, Patrizia, I-10129 Torino (IT); DI SCIPIO, Federica, I-10124 Torino (IT); LOMBARDI, Antonio, I-10024 Moncalieri (TO) (IT)
(74) Representative: Freyria Fava, Cristina

(57) **Abstract**

The present description concerns a composition comprising resveratrol and cyclodextrins for use in preventing dental caries and in a cosmetic method for preventing and/or reducing teeth yellowing and bad breath.

## Description

### Field of the invention

The disclosure concerns a composition for use in the prevention of dental caries and in a cosmetic method for preventing teeth yellowing and bad breath.

### Background of the invention

The oral health in infants and children up to 5 years of age depends, first of all, on the parents and, secondly, on how the dentist provides them with the correct information to maintain it over the time. Indeed, it is necessary to teach how to intervene and anticipate all the possible problems depending on the age, stages of growth and development of the child. The American Academy of Pediatric Dentistry recommends to check children from the age of 6 months or after the eruption of the first tooth, however, no later than one year of age, so to act promptly before that serious oral health problems could arise.

Dentists and/or dental hygienists have to examine the oral cavities of infants and young children to identify tooth decay, dental biofilm, gingivitis, oral pathologies and signs of any orofacial trauma and/or abuse. Furthermore, they have to motivate parents to be informed and attentive to the oral needs of their own children. The insistence on the early prevention, the identification of problems and the need for a reference dentist should improve oral health, reduce pain and decrease the need for next therapeutic interventions.

After having carried out a first general examination of the young patient, the specialist should explain to the parents how important is firstly the removal of the bacterial plaque through the teaching of home oral hygiene maneuvers, as the bacterial plaque is the main responsible for several problems affecting the oral cavity [1,2].

Indeed, the oral cavity is a warm and humid environment able to support the proliferation of a wide spectrum of microorganisms that can easily colonize the mucous and dental surfaces in the mouth, forming three-dimensional, multispecies and structurally organized communities, called biofilms. Biofilms have been defined as microbial populations incorporated in a matrix, adhering to each other and/or to surfaces or interfaces: those that are formed on the teeth constitute the dental plaque and the oral cavity is the only site of the body that has surfaces non-flaking that favor microbial colonization. Inside the biofilm, the bacteria are protected, located in an environment where they have a greater availability of metabolic factors, and can therefore proliferate exponentially. Dental plaque is classified according to its position with respect to the gingival margin in supragingival and subgingival. In the supragingival location (i.e. coronally at the edge of the gingiva), the plaque is easily detectable both visually and with diagnostic tools and has a yellowish-white appearance, concentrating especially in areas not daily cleaned by the patient. It consists of about 80% of water and the remaining 20% of a solid component, in which 70-80% is represented by cellular elements of various nature (corpuscular part) and for 20-30% by intercellular matrix. In the corpuscular part there are essentially bacteria of various morphology, such as cocci, rods and filamentous bacteria. The intermicrobial matrix contains an inorganic part (Ca, P, F, Na, Mg) which is linked to an organic component in a small percentage. The percentage of inorganic component increases as the plaque proceeds in the mineralization process to turn finally into tartar. The organic component (about 70% proteins and carbohydrates, 15% of lipids, and 15% of components of various kinds, such as muramic acid) basically derives from the lysis of the bacteria themselves [3].

The classification of many oral diseases is based on the presence of the bacterial plaque and factors that modify the inflammatory state of the gum. In fact, local or systemic factors can induce the alteration of plaque-induced inflammation. Local factors include anatomical conditions of the tooth, dental restorations, malpositions or orthodontic appliances, while systemic factors involve pathologies of the endocrine system, taking medications, malnutrition or flawed habits such as cigarette smoking. In particular, plaque-induced gingivitis is a reversible gingival inflammation sustained by the bacteria located on the gingival margin, and then spread to the rest of the gum. Epidemiological data indicate that plaque-induced gingivitis is prevalent in toothed populations of all ages and that it is the most common form of periodontal disease. Especially in children, the incidence and prevalence of plaque-induced gingivitis continue to increase until it reaches its peak during puberty [4]. Early changes in gingival health in plaque-induced gingivitis are not always clinically detectable; however, as plaque-induced gingivitis progresses to more advanced forms of the disease, the signs and symptoms become more evident. The normal clinical outcomes of plaque-induced gingivitis include erythema, edema and bleeding, while no clinical attachment loss is revealed by radiographic analysis. If left untreated, it can develop into periodontitis, a pathology characterized by the irreversible destruction of the tooth support apparatus.

Epidemiological data on a representative sample of 12 years old Italian children indicate that the 23.8% of the population has gingival bleeding, while the 28.7% have tartar. Therefore, the 52.5% of the population is not in a state of gingival health and this is due to the lack of a sufficient level of oral hygiene. This epidemiological framework requires the application of preventive educational strategies aimed at learning correct oral hygiene maneuvers [5] .

The effective removal of plaque from dental surfaces depends on numerous factors including the patient's motivation, the knowledge available to him and his manual skills. Hence, it is important to focus the professional oral hygiene sessions on the motivational interview with the young patient and the parents, through information and instruction on the mechanical and chemical home control of plaque.

According to the ministerial guidelines, the correct brushing of the teeth prevents gingivitis and all pathologies associated with bacterial plaque in pediatric patients, and the first oral hygiene methods must be early implemented with the eruption of the first deciduous tooth.

Oral hygiene maneuvers must be carried out by the parent at least twice a day, with the help of small-sized soft bristle brushes. The oral hygiene of children must therefore be left to the parents or supervised by them, until the acquisition of sufficient manual skills: adequate plaque removal requires an effective brushing method obtained thanks to good manual skills. It is thus necessary that the parent personally carries out brushing in the first years of life and supervises it until the child's manual skills are sufficient to guarantee valid brushing.

Moreover, for all the subjects at high risk of gingivitis, additional preventive both home and professional measures are also provided. The reduction in the amount of plaque and the improvement of gingival health can be more achieved by the use of an electric toothbrush with rotating-oscillating movement compared to that obtained by the use of a manual toothbrush. In addition, the use of the electric toothbrush can improve patient compliance with oral hygiene maneuvers or in patients who have poor manual skills or inability to maintain an adequate level of oral hygiene with the manual toothbrush. However, the use of the electric toothbrush is only recommended in children over the age of 5 years: for younger children it would be desirable to associate manual brushing with a pharmacological treatment, although there are no safe active ingredients that can be used in such small patients [5,6].

The Guidelines of the Italian Ministry of Health state that chemical plaque control may be necessary in those pediatric patients who are unable to adequately control supragingival biofilm with mechanical devices. Therefore, the use of chemicals should be additive to that of mechanical devices, allowing a more effective action to reduce the amount of biofilm. The use of antiseptics for the control of plaque and gingivitis have been widely evaluated in the literature: in several systematic reviews, the effectiveness of some formulations has been observed, however associated to side and undesirable effects [7].

The inhibitory effect of chlorhexidine (CHX) on the formation of bacterial plaque is widely documented in the literature. CHX is considered as the "gold standard" of oral antiseptics for its antimicrobial activity, low toxicity and high substantivity [8]. It is frequently used in all clinical pictures that interrupt oral hygiene maneuvers. Nevertheless, CHX can lead to numerous side effects, e.g. the pigmentation of the dental surfaces and the mucous membranes, the temporary loss and alteration of the perception of tastes and flavor, the irritation of the mucous membranes, the dryness of the mouth and, in the most serious cases, the swelling of the parotid glands. All these possibilities have a strong negative impact on patient compliance and do not allow clinicians to use CHX for pediatric patients younger than 6 years [7]. It is therefore difficult to find products suitable for the chemical control of plaque in these young patients, not only for the aforementioned problems, but also because at this age, children are not able to perform rinses without swallowing the mouthwash, with possible toxic consequences on the gastrointestinal apparatus.

For these reasons, the attention of many researchers has turned to molecules of natural origin characterized by good anti-inflammatory/antimicrobial activities with less adverse effects [8]. Unfortunately, the very encouraging preclinical results obtained *in vitro* on various cellular models have not been confirmed *in vivo.* Their chemical structures, in fact, have adversely affected their pharmacological properties: low water solubility, and rapid metabolization with consequent inactivation (i.e. low bioavailability) are the main causes of therapeutic failure of these molecules.

Resveratrol (RV) has aroused great interest for its remarkable pleiotropic activities (anti-inflammatory, anti-bacterial, anti-oxidant, chemopreventive) [9]. RV is a phytoalexin produced by many plants for protective purposes, in response to infections (viral, bacterial, fungal), but also to environmental stresses (UV rays, water stresses). It was originally isolated in 1940 from the roots of white hellebore and more recently from many other plant species, including *Polygonum cuspidatum,* a healing plant with an important role in Japanese and Chinese folk medicine. It is also present in *Vitis vinifera* (common vine) and, consequently, also in wine (especially red). RV is found both in the form of free species and in the glycosylated form, the 3β-glucoside of RV. Chemically, RV is a non-flavonoid polyphenolic compound, of the stilbenic type, with a skeleton composed of two aromatic rings joined by a methylene bridge on which 3 hydroxyl groups are grafted in position 3, 5 and 4'. Thanks to them, the RV is able to form a radical derivative stabilized by the electronic delocalization on the two aromatic rings and on the methylene bridge that connects them, thus obtaining the antioxidant properties typical of polyphenols. The stilbenic compounds are widely represented in nature and include a wide range of molecules: from January 1995 to the end of 2015, more than 400 new stilbenes have been isolated and identified [10]. They have a C6-C2-C6 structure and are considered among the main natural non-flavonoid compounds of food and potentially therapeutic importance (C6-C1 phenolic acids) [11]. Stilbenes are secondary metabolites (with molar masses of about 200-300 g/mol) of numerous plants. They have the basic 1,2-diphenylethylene structure and exist as two different stereoisomers in E- (or trans) and Z- (or cis) forms, depending on where the functional groups are linked to each other on both sides of the double bond. These two configurations have been shown to be responsible for different pharmacological activities: the cis-structures have predominantly antimitotic activity, while the transforms are powerful inhibitors of the activity of enzymes such as DNA polymerase and cyclooxygenase (COX) [12].

The obtained results in recent years, from the numerous experimental models used, have suggested that RV could prove to be an important agent in the prevention of pathological processes such as inflammation, atherosclerosis and carcinogenesis.

In particular, it has been shown to possess *in vitro:*
- Anti-oxidant/anti-inflammatory activity;
- Anti-bacterial activity;
- Protective activity against cardiovascular diseases;
- Anti-neoplastic activity;
- Activities on aging;
- Activity on bone metabolism.

As with other plant-based molecules, unfortunately, the very encouraging preclinical results obtained *in vitro* have not been confirmed *in vivo:* the data on its clinical efficacy have always been unsatisfactory, failing the end-points of the clinical trials carried out [13]. Despite this, it is one of the most advertised polyphenols and proposed as an antioxidant supplement.

There is therefore still the need of compositions comprising natural compounds that can be useful for preventing dental caries and that can be used also by a pediatric population.

### Summary of the invention

The main object of this disclosure is to provide a composition useful for preventing dental caries that can be used by all the human population, preferably the pediatric one (with age comprised between 1 and 5 years).

A further object is to provide a cosmetic method for preventing and/or reducing teeth yellowing and bad breath.

According to the invention, the above object is achieved thanks to the subject matter recalled specifically in the ensuing claims, which are understood as forming an integral part of this disclosure.

The present invention provides a composition comprising resveratrol and cyclodextrins for use in preventing dental caries.

The present invention further provides a cosmetic method for preventing and/or reducing teeth yellowing and bad breath comprising application of a composition containing resveratrol and cyclodextrin to teeth and oral cavity.

### Brief description of the drawings

The invention will now be described in detail, purely by way of illustrative and non-limiting example, with reference to the attached figures, wherein:
- **Figure 1****:** Full Mouth Plaque Score of the RV and of the control groups at T0, T1, T2 (*** p< 0.001).
- **Figure 2****:** Full Mouth Bleeding Score of the RV and of the control groups at T0, T1, T2 (*** p< 0.001).

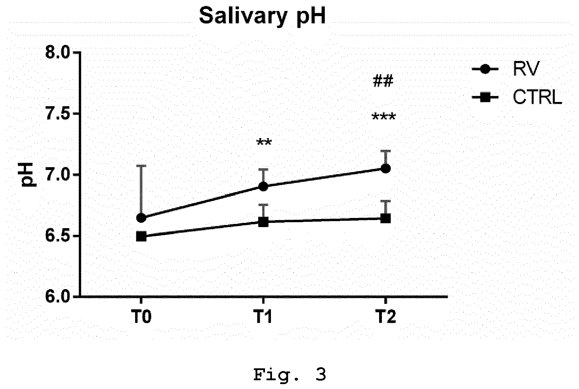

- **Figure 3****:** Salivary pH of the RV and of the control groups at T0, T1, T2 (** p=0.045 (T0 vs T1); *** p< 0.001 (T0 vs T2); ## p=0.045 (T1 vs T2)).

### Detailed description of the invention

The invention will now be described in detail, by way of non-limiting example, with reference to a novel use of resveratrol complexed with cyclodextrins for the prevention of dental caries in a pediatric population. It is clear that the scope of this description is in no way limited to this specific population, since also an adult population can get benefit from the use of such a composition for preventing dental caries.

In the following description, numerous specific details are given to provide a thorough understanding of embodiments.

The embodiments can be practiced without one or more of the specific details, or with other methods, components, materials, etc. In other instances, well-known structures, materials, or operations are not shown or described in detail to avoid obscuring aspects of the embodiments.

Reference throughout this specification to "one embodiment" or "an embodiment" means that a particular feature, structure, or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The headings provided herein are for convenience only and do not interpret the scope or meaning of the embodiments.

In one embodiment, the present disclosure concerns a composition comprising resveratrol and cyclodextrin for use in preventing dental caries in a subject, preferably a pediatric subject, more preferably a pediatric subject with age comprised between 1 and 5 years. The composition object of the present disclosure is free of any other active principle(s) with anti-caries activity and/or any active principle(s) with a salivary pH regulator activity.

In one preferred embodiment, the composition essentially consists - in terms of active principles necessary for the prevention of dental caries - of resveratrol complexed with cyclodextrins.

In one embodiment, the composition is an aqueous formulation.

In one embodiment, the composition contains resveratrol in an amount (weight/volume) comprised between 0.200% and 0.5%, preferably between 0.28% and 0.48%, more preferably between 0.3% and 0.35% with respect to the volume of the composition.

In one embodiment, the composition contains cyclodextrin in an amount (weight/volume) comprised between 0.2% and 0.5%, preferably between 0.28% and 0.48%, more preferably between 0.3% and 0.35% with respect to the volume of the composition.

In one embodiment, the cyclodextrin is selected from α-cyclodextrin, β-cyclodextrin, and γ-cyclodextrin, preferably β-cyclodextrin, and γ-cyclodextrin, more preferably β-cyclodextrin.

In one embodiment, the cyclodextrin has a molecular weight comprised between 1125 g/mol and 2020g/mol, preferably between 1130 g/mol and 1500 g/mol.

In one embodiment, the composition further contains at least one aroma, and/or at least one excipient. The at least one aroma is selected from lemon, black cherry, strawberry, pear, banana, cola, orange, peppermint, licorice, berries, apple, blueberry, vanilla. The at least one excipient is selected from colorants, sweeteners, preservatives, substances to make the product more palatable (e.g. glycerin and sorbitol).

In one embodiment, the composition is in form of mouthwash, or spray.

In one embodiment, the subject is an adult or a pediatric subject, preferably a pediatric subject, more preferably a pediatric subject with age comprised between 1 and 5 years.

Dental caries results from the absence of an adequate control of the bacterial plaque, and the presence of acidogenic species within the biofilm generated by the bacteria.

Dental caries is one of the most common degenerative process that damages the hard tissues of the tooth, mainly caused by bacteria present in the oral cavity. They take refuge in the plaque, a patina adhering to the surface of the teeth that originates from the precipitation of proteins and salivary mucoids: in similar circumstances, the bacteria adhere to it and proliferate. Moreover, a salivary pH near to neutrality is an important indicator of the health of the oral cavity. Positive and negative variations are quite frequent (caused by the type of diet, the degree of oral hygiene and pathological factors), but regardless of the cause, an acid salivary pH is an important risk factor for caries, the erosion of dental enamel and dentinal hypersensitivity, also causing important qualitative and quantitative variations within the biofilms of the dental plaque.

Thus, the control of plaque and saliva pH consequently leads to the control of the formation of dental caries.

The mechanical control of the plaque can be an effective strategy for the prevention and for the treatment of dental caries both in an adult population and in a pediatric one (between 2 and 5 years of age). However, it is not enough and it has to be addressed also with chemical treatments that contribute to reduction of dental plaque and to the restoration of the correct salivary pH.

The aim of the randomized double-blind pilot study in a pediatric population, described in the following, was to evaluate the effectiveness of a mouthwash comprising resveratrol (RV) complexed with cyclodextrins (CDs) in terms of reduction of plaque accumulation, consequent gingival inflammation, possible pigmentation of dental surfaces, improvement of salivary pH and experience of the patient and parents in using it.

The statistical analysis of the study showed that:
1. the experimental and control groups are equivalent in gender, age and size distribution;
2. no differences were found between the three clinical parameters (Full Mouth Plaque Score (FMPS), Full Mouth Bleeding Score (FMBS), pH) of the two groups before starting the study (i.e. at T0);
3. the pH was significantly less acidic between T0 and the end of the study (i.e. at T2) for experimental group, which also showed a significant difference between T0 and an intermediate time of evaluation (indicated as T1) and between T1 and T2;
4. there were significant reductions in bacterial plaque and bleeding between T0 and T1, between T1 and T2, and overall between T0 and T2 in the experimental group;
5. in the comparison of the three parameters in the two groups at T1, the experimental group showed a greater reduction in both the plaque index and the bleeding and the patients had normal pH range for saliva (6.8-7.4), respect to the control group; at T2, the experimental group showed less development of bacterial plaque and less bleeding, with an average of FMPS of 10%, an average of FMBS of 2% and pH values around 7, ideal for guaranteeing gingival health;
6. in all the patients of the experimental group, no pigmentations of the dental surfaces were detected due to the use of the spray mouthwash, which was appreciated both in taste and for ease of administration, demonstrating a good compliance.

In view of the evidence provided herein (i.e. reduction in the development of bacterial plaque and neutralization of the salivary pH in the experimental group), it emerges that the composition comprising resveratrol complexed with cyclodextrins is useful in preventing formation of dental caries both in an adult and in a pediatric population.

In a further embodiment, the present disclosure concerns a cosmetic method for preventing and/or reducing teeth yellowing and bad breath comprising application of a composition containing resveratrol and cyclodextrin to teeth and oral cavity. Preferably, the composition essentially consists - in terms of active principles necessary for preventing and/or reducing of teeth yellowing and bad breath - of resveratrol complexed with cyclodextrin.

In one embodiment, the composition used in the cosmetic method is an aqueous formulation, preferably in form of a mouthwash, or spray.

In one embodiment, the composition used in the cosmetic method contains resveratrol in an amount (weight/volume) comprised between 0.200% and 0.500% with respect to the volume of the composition, preferably between 0.280% and 0.480%, more preferably between 0.300% and 0.350%.

In one embodiment, the composition used in the cosmetic method contains cyclodextrin in an amount (weight/volume) comprised between 0.200% and 0.500% with respect to the volume of the composition, preferably between 0.280% and 0.480%, more preferably between 0.300% and 0.350%. Preferably the cyclodextrin is selected from α-cyclodextrin, β-cyclodextrin, and γ-cyclodextrin, more preferably the cyclodextrin is selected from β-cyclodextrin, and γ-cyclodextrin, still more preferably the cyclodextrin is β-cyclodextrin.

In one embodiment, the composition used in the cosmetic method further contains at least one aroma, and/or at least one excipient.

It is in fact known that inadequate brushing, flossing, and rinsing with an antiseptic mouthwash to remove plaque and stain-producing substances, like coffee and tobacco, can cause tooth yellowing and bad breath. Indeed, in addition to bad eating habits to maintain the natural shade of the teeth (e.g. coffee, tea, licorice, chocolate, red wine, sodas), yellowing can be a consequence of plaque and tartar deposits.

In view of the experimental data provided herein (see above and the section "Results"), it emerges that the composition comprising resveratrol complexed with cyclodextrins represents a good tool for controlling teeth yellowing and bad breath.

### METHODS

### Composition

The mouthwash used in the clinical trial is an aqueous formulation containing resveratrol (purchased from Fagron Italia) in an amount of 0.318% w/v, β-cyclodextrin (purchased from Roquette) in an amount of 0.318% w/v, strawberry liquid aroma in an amount of 0.1% w/v and deionized water in an amount of 93.764% w/v with respect to the final composition. The mouthwash then contains sodium benzoate (in an amount 0.3 % w/v), glycerin (in an amount 5.0 % w/v), and sodium saccharine (in an amount 0.2 % w/v).

The RV mouthwash is manufactured as follows.

First of all, resveratrol and β-cyclodextrin are mixed together in a clean and sterile recipient to produce a so called phase A. A dissolver is introduced into the recipient and operated to shake at low speed without swirling for several hours; after, water is added to dilute the complex (resveratrol and β-cyclodextrin) to obtain a so called phase B; subsequently, sodium benzoate, glycerin, sodium saccharin, liquid aroma are added together to obtain a so called phase C that is then added to the phase B. Always under agitation, the suspension is mixed for about 10 to 15 min. The solution obtained is filtered and then packaged.

The RV mouthwash in spray form is obtained by packaging the liquid mouthwash in a bottle provided with a spray solubilizer.

### Patient population

Fifty-eight subjects with diffuse bacterial plaque and gingival inflammation were chosen among the patients of the Pediatric Department in the Dental School of Turin during their first dental visit. All the enrolled subjects were aged between 2-5 years, they had to have a plaque index greater than 15% (Full Mouth Plaque Score> 15%) and bleeding on the periodontal survey. These patients followed a hygiene protocol including the use of the mouthwash described above between one session and another. The data obtained in these subjects were compared with those of a control group, already treated in the past at the Dental School's Department of Pediatric Dentistry, with the same identical characteristics and inclusion criteria. These patients had followed the same hygiene protocol, but without the use of any mouthwash.

The following were included in the study:
- Age between 2 and 5 years (2 and 5 years included);
- FMPS (Full Mouth Plaque Score) > 15 % at baseline (T0);
- FMBS (Full Mouth Bleeding Score) > 0% at baseline (TO) .

The following were not included in the study:
- Patients with systemic pathologies or syndromes;
- Patients treated with drugs causing as a side effect an increase in gingival volume;
- Patients with known intolerance or allergy to any component of the mouthwash in the study.

### Procedures of the study

Three sessions were agreed with the parents of the patients, 15 days apart.

*First visit (T0-Baseline):* for each patient a medical record was compiled. Some information was collected:
1. the remote medical history;
2. the immediate medical history;
3. the dental history of the child.

Subsequently, all patients underwent a dental examination by an operator, in which an extraoral and an intraoral examination were performed. All the following data were recorded:
- any anomalies at the level of the face, the mucous membranes, and the salivary glands;
- the presence of dental anomalies, such as the presence of supernumerary teeth, or any agenesis;
- the presence of carious lesions: their prevalence in the subject's oral cavity were calculated, according to the criteria established by the World Health Organization (WHO) giving each patient a score by the sum of the decayed teeth, missing (by extraction or trauma) and completed with the Decay-missing-filled (dmft) index for deciduous dentition.

During the intraoral examination the following parameters were also detected:
- the presence of bacterial plaque on the dental surfaces and calculation of the Full Mouth Plaque Score;
- evaluation of the degree of gingival inflammation in relation to the presence of bleeding and calculation of the Full Mouth Bleeding Score;
- evaluation of the pigments on the tooth surface by means of the Stain Index.

Once the data collection was complete, parents were instructed on home oral hygiene maneuvers to be performed on the child, twice a day in the morning and in the evening: tooth brushing with Fones (i.e. circular brushing) technique using a manual toothbrush and Elmex toothpaste for children 0-6 years.

The mouthwash in spray solution were applied once a day (in the evening 30 minutes after brushing the teeth) at the level of the patient's gingival margin both on the vestibular and on the lingual/palatal side of all the gums.

*Second visit (T1):* each patient was re-evaluated 15 days after the first visit. The bleeding parameters, the presence of plaque and spots already assessed on the first time were recorded again, in order to compare them. A *polish* was performed as a professional oral hygiene maneuver for the total elimination of bacterial plaque. At the end of the session, the mouthwash was prescribed again for further 15 days, with the same indications described in T0 to evaluate the control of the plaque by the patient, after the complete elimination of it.

*Third visit (T2):* each patient underwent a visit after 15 days in order to detect and evaluate, for the last time, the parameters of bleeding, the presence of plaque and spots and to submit a questionnaire to the parents and the patients of appreciation on the use of mouthwash.

### Statistical Analysis

The trend of the clinical parameters was analyzed considering the patient as the statistical unit of reference. Data were expressed as means ± standard or median deviation and interquartile range (quantitative variables) or as frequency distribution (categorical variables). Through the Kolmogorov-Smirnov test and the Shapiro-Wilk test the Gaussian distribution of the quantitative variables were analyzed. For the intra-group analysis of the variations over time of the clinical variables, the analysis of variance for repeated measures (quantitative parametric variables) or Friedman's test (non-parametric quantitative variables) followed by post-hoc tests were used. For intergroup analysis the statistical significance of the differences between the test group and the control group were analyzed by means of the chi-square test (categorical variables), T-Student test for unpaired data (parametric quantitative variables) or Mann test-Whitney (non parametric quantitative variables). Bonferroni's correction was used for multiple comparisons. A significance level of 5% were considered.

### RESULTS

### Clinical Results

Fifty-eight subjects aged between 2 and 5 years participated in the above study. RV-based mouthwash to be associated with mechanical plaque control was supplied to 29 patients (RV group), while the remaining 29 were only provided with instructions on mechanical control of plaque (Control group). All patients were evaluated in three sessions (T0 - T1 - T2), each at a distance of 15 days from each other, and during the period they were not controlled. Tables 1 and 2 summarize the demographic characteristics of the sample; there are no statistically significant differences for these variables in the two patient groups.

**Table 1**

| | **Average age** | **No.** | **Std. Dev.** |
|---|---|---|---|
| **RV group** | 3.62 | 29 | 1.05 |
| **Control group** | 3.62 | 29 | 0.97 |

**Table 2**

| **Sex** | **No.** | **Percentage in treatment** |
|---|---|---|
| **Female** | 15 | 51.7 |
| **Male** | 14 | 48.3 |

There was no significance between FMPS, FMBS parameters of the two groups, recorded at T0: p FMPS = 0.06; p FMBS = 0.849 (Table 3).

**Table 3**

| | **Group** | **No.** | **Media** | **Standard deviation** |
|---|---|---|---|---|
| **FMPS - Baseline** | RV group | 29 | 61.41 | 14.01 |
| | Control group | 29 | 57.86 | 15.25 |
| **FMBS - Baseline** | RV group | 29 | 29.03 | 10.89 |
| | Control group | 29 | 29.62 | 12.45 |

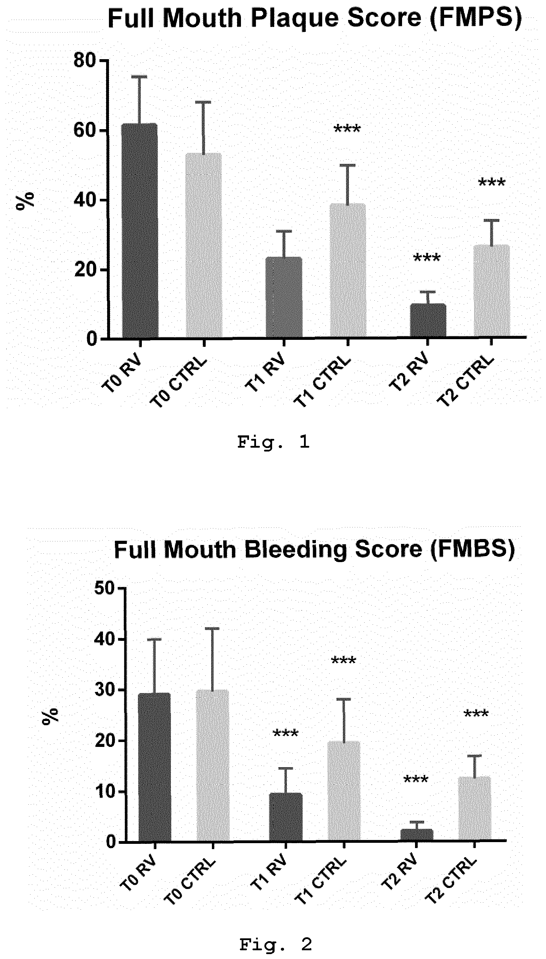

FMPS and FMBS were assessed at the different times, T0, T1, T2. Both in RV and CONTROL groups, both FMPS (Figure 1) and FMBS (Figure 2) improve significantly between T0 and T1, T1 and T2, and overall between T0 and T2 with a p <0.001.

As regards the comparison of the salivary pH between the two groups, no significance was identified at baseline T0 (p = 0.299) . On the contrary, in the CONTROL group, the pH values approached to neutrality only in the comparison between T0 vs T1 (p = 0.021), while in the RV group, there was a statistically significant improvement in all timings, both between T0 vs T1 (p = 0.045) and between T1 vs T2 (p = 0.045) and overall between T0 vs T2 (p <0.001).

At T1, 15 days after starting the treatment, in the RV group, there was a greater reduction in plaque and in bleeding, and an improvement in pH compared to the CONTROL group (p <0.001) (table 4).

**Table 4**

| | | **FMPS at 15 days** | **FMBS at 15 days** | **pH at 15 days** |
|---|---|---|---|---|
| **RV group** | **No.** | 29 | 29 | 29 |
| | **Media** | 22.93 | 9.27 | 6.90 |
| | **Std. Dev.** | 7.91 | 5.18 | 0.18 |
| | **Median** | 21 | 8 | 7 |
| **Control group** | **No.** | 29 | 29 | 29 |
| | **Media** | 38.06 | 19.34 | 6.61 |
| | **Std. Dev.** | 11.66 | 8.70 | 0.25 |
| | **Median** | 36 | 18 | 6.6 |

At T2, 30 days after starting the treatment and 15 days after the professional oral hygiene, it is evident that the RV group has less bacterial plaque, with less bleeding and a salivary pH stable at neutral values, with a p <0.001 for all three clinical parameters (Table 5).

**Table 5**

| | | **FMPS at 30 days** | **FMBS at 30 days** | **pH at 30 days** |
|---|---|---|---|---|
| **RV group** | **No.** | 29 | 29 | 29 |
| | **Media** | 9.27 | 2.03 | 7.22 |
| | **Std. Dev.** | 3.9 | 1.70 | 2.3 |
| | **Median** | 9 | 2 | 7 |
| **Control group** | **No.** | 29 | 29 | 29 |
| | **Media** | 26.10 | 12.27 | 6.58 |
| | **Std. Dev.** | 7.57 | 4.49 | 1.02 |
| | **Median** | 26 | 12 | 6.6 |

### Subjective evaluation

Patients' parents were requested to fill in a satisfaction questionnaire on the use of RV-based mouthwash. From the 29 completed questionnaires, it was revealed that all parents were satisfied with the use of RV-based mouthwash, they had no difficulty in administering it to the child, thanks to the spray formulation; as far as children are concerned, they never opposed its use and everyone liked the strawberry taste: this aspect was also confirmed by the parents.

### Bibliography

1. Casamassimo, P. (1996): Bright Futures in Practice: Oral health. Arlington, VA, National Center for Education in Maternal and Child Health, pp 1-41.
2. Casamassimo P.S. and Warren, J.J (2005): Examination, Diagnosis and Treatment Planning of the Infant and Toddler, Pediatric Dentistry: Infancy Through Adolescence, 4th ed. St. Louis, Elsevier Saunders, pp 208-214.
3. Marsh, P. (2005). Dental plaque: biological significance of a biofilms and community life-style. Journal of Clinical Periodontology, pp 7-15.
4. Stamm, J.W. (1986). Epidemiology of gingivitis. Journal of Clinical Periodontology 13, 360-366.
5. Deacon, S.A., Glenny, A.M., Deery, C. et al. (2010). Different powered toothbrushes for plaque control and gingival health. Cochrane Database of Systematic Reviews, CD004971.
6. Addy, M., Moran, J. Evaluation of oral hygiene products: science is true; don't be misled by the facts. Periodontology 2000, 1997;15:40-51.
7. Jones, C.G. Chlorhexidine: is it still the gold standard? Periodontol 2000, 1997;15:55-62.
8. Van Strydonck, D.A., Slot, D.E., Van der Velden, U., Van der Weijden, F. Effect of a chlorhexidine mouthrinse on plaque, gingival inflammation and staining in gingivitis patients: a systematic review. J Clin Periodontol 2012; 39:1042-1055.
9. Jang, M., Cai, L., Udeani, G.O., Slowing, K.V., Thomas, C.F., Beecher, C.W., Fong, H.H., Farnsworth, N.R., Kinghorn, A.D., Mehta, R.G., Moon, R.C., Pezzuto, J.M. Cancer chemopreventive activity of resveratrol, a natural product derived from grapes. Science. 1997 Jan;275(5297):218-20.
10. Shen, T., Wang, X.N., Lou, H.X. Natural stilbenes: an overview. Nat Prod Rep. 2009 Jul;26(7):916-35.
11. Crozier, A., Jaganath, I.B., Clifford, M.N. Dietary phenolics: chemistry, bioavailability and effects on health. Nat Prod Rep. 2009 Aug;26(8):1001-43.
12. Roupe, K.A., Remsberg, C.M., Yáñez, J.A., Davies, N.M. Pharmacometrics of stilbenes: seguing towards the clinic. Curr Clin Pharmacol. 2006 Jan;1(1):81-101.
13. Amri, A., Chaumeil, J.C., Sfar, S., Charrueau, C. Administration of resveratrol: What formulation solutions to bioavailability limitations? J Control Release. 2012 Mar 10;158(2):182-93.

## Claims

1. A composition comprising resveratrol and cyclodextrin for use in preventing dental caries in a subject.

2. The composition for use according to claim 1, wherein the composition is an aqueous formulation.

3. The composition for use according to claim 1 or claim 2, wherein the composition contains resveratrol in an amount comprised between 0.200% and 0.500% (w/v).

4. The composition for use according to any one of the preceding claims, wherein the composition contains cyclodextrin in an amount comprised between 0.200% and 0.500% (w/v) .

5. The composition for use according to any one of the preceding claims, wherein the cyclodextrin is selected from α-cyclodextrin, β-cyclodextrin, and γ-cyclodextrin.

6. The composition for use according to any one of the preceding claims, wherein the cyclodextrin has a molecular weight comprised between 1125 g/mol and 2020 g/mol.

7. The composition for use according to any one of the preceding claims, wherein the composition further contains at least one aroma, and/or at least one excipient.

8. The composition for use according to any one of the preceding claims, wherein the composition is in form of liquid mouthwash, spray.

9. The composition for use according to any one of the preceding claims, wherein the subject is an adult or a pediatric subject, preferably a pediatric subject aged between 1 and 5 years old.

10. A cosmetic method for preventing and/or reducing teeth yellowing and bad breath comprising application of a composition containing resveratrol and cyclodextrin to teeth and oral cavity.

11. The cosmetic method according to claim 10, wherein the composition is an aqueous formulation, preferably in form of a liquid mouthwash, spray.

12. The cosmetic method according to claim 10 or claim 11, wherein the composition contains resveratrol in an amount comprised between 0.200% and 0.500% (w/v).

13. The cosmetic method according to any one of claims 10 to 12, wherein the composition contains cyclodextrin in an amount comprised between 0.200% and 0.500% (w/v).

14. The cosmetic method according to any one of claims 10 to 13, wherein the cyclodextrin is selected from α-cyclodextrin, β-cyclodextrin, and γ-cyclodextrin.

15. The cosmetic method according to any one of claims 10 to 14, wherein the composition further contains at least one aroma, and/or at least one excipient.
